(19) **Europäisches Patentamt**
**European Patent Office**
**Office européen des brevets**

(11) **EP 2 823 829 B1**

(12) **EUROPEAN PATENT SPECIFICATION**

(45) Date of publication and mention
of the grant of the patent:
**20.07.2016  Bulletin 2016/29**

(51) Int Cl.:
*A61L 27/22* (2006.01)
*A61K 38/29* (2006.01)
*A61K 38/48* (2006.01)
*A61K 33/06* (2006.01)
*A61L 27/50* (2006.01)
*A61K 33/00* (2006.01)
*A61P 19/08* (2006.01)
*A61K 38/36* (2006.01)
*A61K 45/06* (2006.01)
*A61L 27/44* (2006.01)
*A61K 33/42* (2006.01)

(21) Application number: **14003058.6**

(22) Date of filing: **18.04.2008**

(54) **Fibrin compositions containing strontium compounds for use in bone growth**

Fibrinzusammensetzungen mit Strontiumverbindungen zur Knochenbildung

Compositions à base de fibrine contenant des composés de strontium pour stimuler l'ossification

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **23.04.2007  US 925716 P**

(43) Date of publication of application:
**14.01.2015  Bulletin 2015/03**

(62) Document number(s) of the earlier application(s) in accordance with Art. 76 EPC:
**08746189.3 / 2 142 224**

(73) Proprietors:
• **Baxter International Inc.**
**Deerfield, IL 60015 (US)**
• **Baxter Healthcare SA**
**8152 Glattpark (Opfikon) (CH)**

(72) Inventors:
• **Barry, John J.**
**1100 Vienna (AT)**
• **Goessl, Andreas**
**1180 Vienna (AT)**
• **Zimmer, Gerald**
**3364 Neuhofen/Ybbs (AT)**

(74) Representative: **Müller-Boré & Partner**
**Patentanwälte PartG mbB**
**Friedenheimer Brücke 21**
**80639 München (DE)**

(56) References cited:
**EP-A- 1 741 436          WO-A-2005/082385**
**US-A- 5 618 549          US-A- 5 856 356**
**US-A1- 2006 148 704      US-A1- 2006 216 321**

• **MARIE P J ET AL: "AN UNCOUPLING AGENT CONTAINING STRONTIUM PREVENTS BONE LOSS BY DEPRESSING BONE RESORPTION AND MAINTAING BONE FORMATION IN ESTROGEN-DEFICIENT RATS", JOURNAL OF BONE AND MINERAL RESEARCH, NEW YORK, NY, US, vol. 8, no. 5, 1 May 1993 (1993-05-01), pages 607-615, XP000646111, ISSN: 0884-0431**

**Description**

The present invention relates to a composition for use in bone healing or bone growth comprising fibrinogen, thrombin and a strontium (Sr) containing compound, wherein strontium is present in a 25% strontium substituted calcium salt.

The current practice in bone healing and regeneration is to fill bone voids with either a bone graft (auto or allograft), a bone cement such as polymethylmethacrylate (PMMA) or injectable or implantable calcium salt void fillers. Autografts are the 'gold standard' choice for this application but there are issues with donor tissue limitations, trauma, infection and morbidity. There are a number of problems with allografts, including the risk of disease transmission and imunogenicity. Both auto and allografts display loss of biological and mechanical properties due to secondary remodeling. It is these limitations that have prompted interest in alternative materials to bone grafts (Parikh SN. 2002; J. Postgrad. Med. 48:142- 148)

PMMA is a non-resorbable polymeric material. During its polymerization unreacted monomer, catalyst and low molecular weight oligomers become entrapped in the polymer. These chemicals have the potential to leach out of the material resulting in localised cytotoxic and immunological responses. PMMA polymerization has a high exotherm that can potentially cause heat necrosis. This exotherm also limits the ability of PMMA to incorporate any pharmacological or chemotherapeutic agents. PMMA leakage from a defect can result in very serious complications including compression of adjacent structures (requiring further surgery) and/or embolism.

Bone matrix at the nano-level ($10^{-9}$ m) is composed of collagenous fibers in which tiny crystals of calcium salts are embedded. In order to simulate the natural make up of bone, calcium salts are frequently used to repair bone. There are a number of known calcium salt based "injectable void fillers." One major complication with calcium salt cements is their requirement for setting *in vivo* which is usually achieved by chemical reaction. Thus any biologics incorporated in the filler such as cells and pharmacological agents can potentially be damaged. Furthermore if the filler is too "fluid" it can leak out of the defect into adjacent spaces leading to compression of structures and possible emboli. Leakage from defects proximal to joints can potentially impair the joints function.

The calcium phosphate (CP) salt hydroxyapatite (HA structure $Ca_{10}(PO_4)_6(OH)_2$) is closest to the mineral phase of bone and can easily be synthesized at the bench. It is also possible to prepare HA where other cations (for example, magnesium, sodium and strontium) or anions (chloride, fluoride and carbonate) are substituted into the crystal. Relating to the present invention is the substitution of calcium ($Ca^{2+}$) ions with strontium ($Sr^{2+}$) ions in the CP crystals. Strontium and calcium both belong to the alkaline earth elements, and resemble each other in that greater than 99% of the total calcium and strontium in the body is localized in bone. Strontium is the only element identified to have a positive relationship with bone strength, i.e., the mechanical strength of the bone increases with increasing strontium content. The potential therapeutic effects of strontium have been known for some time. However, it has been suggested in the literature that the therapeutic potential may have been overlooked due to confusion of normal, stable $Sr^{2+}$ ($^{84}$Sr, $^{86}$Sr, $^{87}$Sr and $^{88}$Sr) with its radioactive isotopes ($^{85}$Sr, [86m]Sr, $^{87}$Sr and $^{88}$Sr) Dahl et al., Bone; 28 (4) pp. 446-453. Recently, the therapeutic effects of strontium have been highlighted, and research has demonstrated that strontium salts stimulate bone formation and inhibit bone resorption *in vitro* (even at low doses) and *in vivo,* as evaluated by bone histomorphometry in rodents and osteoporotic patients. Ferraro et al, 1983; Calcif Tissue Int. 35:258-60.

The powerful anabolic and antiresorptive effects of strontium has lead to the development of a new anti-osteoporotic drug. The orally active drug (active ingredient strontium ranelate) has been shown to reduce the risk of vertebral fractures and to increase bone mineral density. Meunier PJ et al, 2004; N Engl J Med. 350(5):459-68. The drug is composed of one molecule of ranelic acid bound to two atoms of strontium. The ranelate group was chosen as the carrier because it is tolerated well in the GI tract. In the gut the strontium ions are released from the ranelate group leading to absorption of the strontium by the gut mucosa. Upon absorption, the strontium exhibits strong affinity for bone tissue, and becomes incorporated into the outermost hydrated layer of hydroxyapatite. A smaller amount integrates into the bone crystal, where the ratio of strontium over calcium atoms does not exceed 1:10, so that neither the formation nor the physicochemical properties of the crystal are altered by the presence of strontium. Strontium ranelate is suggested to be the first medication to uncouple bone formation from bone resorption. In vitro, strontium ranelate increases collagen and non-collagen protein synthesis, enhancing pre-osteoblast differentiation, inhibiting osteoclast differentiation, reducing osteoclast function and increases osteoclast apoptosis. In animal models, the increase in bone density is closely correlated with increases in biomechanical bone strength. In rat models, strontium ranelate increases bone mass and improves microarchitecture and bone geometry, resulting in increased bone resistance. In ovariectomized rats (a model for osteoporosis), strontium ranelate decreases bone resorption but maintains high bone formation. All the determinants of bone strength are positively influenced by strontium ranelate treatment (bone mass, dimension, microarchitecture, and intrinsic bone tissue quality). The increment in bone mechanical properties is characterized by an increase in maximal load but also by a dramatic improvement in energy to failure, which is essentially due to an increment in plastic energy. Kendler, 2006; Curr. Osteopor. Rep. 4(1):34-9; Marie, 2006; Curr. Opin. Rheum. 18 Suppl1:S11-5; Ammann, 2006, Bone. 38(2 Suppl 1):15-8. Although the mode of action of the strontium is not fully understood, the increase in osteogenesis is believed not to be associated with changes in circulating levels of 1,25(OH)2 vitamin D or in parathyroid hormone effects. Strontium has been shown anti-fracture efficacy at all sites in a large number of post menopausal women. Close et al, 2006; Expert Opin Pharmacother 7(12):1603-15.

The localized application of strontium in the form of a strontium-containing bone cement has also been investigated by other researchers Wong, Chi-Tak, 2004, "Osteoconduction and osseointegration of a strontium-containing hydroxyapatite bioactive bone cement : in vitro and in vivo investigations. Publisher: University of Hong Kong" (Pokfulam Road, Hong Kong); Zhao et al, 2004; J Biomed Mater Res B Appl Biomater. 69(1):79-86. The nonresorbing Sr-HA bone cement was composed mainly of strontium-containing hydroxyapatite (Sr-HA) filler and bisphenol A diglycidylether dimethacrylate (BIS-GMA) resin. It was proposed that the material could be used for vertebroplasty as it could be delivered by injection and had better physico/mechanical properties than PMMA. The strontium-containing hydroxyapatite (Sr-HA) bone cement was demonstrated to be bioactive both in vitro and in vivo. In-vitro it appeared to be more efficacious than a non-doped hydroxypatite (HA) bone cement for cell attachment (SaOS-2) and bone mineralization. In-vivo the Sr-HA bone cement bonded with natural bone.

In U.S. Patent Nos. 5,736,132 and 5,549,904, a transglutaminase supplemented adhesive tissue adhesive is said to preferably contain a divalent metal ion such as calcium and strontium in an amount in the range of about 0.5 mM to about 100 mM at a pH of about 7.0 to about 8.5. It is claimed that the transglutaminase supplemented adhesive can be used in the treatment of tissue in joint fractures, chondral defects, superficial chondral defects, full-thickness defects, osteochondritis dissecans, meniscal tears, ligament tears, tendon tears, muscle lesions, myotendinous junction lesions, cartilage transplantation, bone transplantation, ligament transplantation, tendon transplantation, chondral transplantation, chondroosseous transplantation, skin graft fixation and blood vessels, patching vascular grafts and microvascular blood vessel anastomosis. The primary focus of the patents is the incorporation of the transglutaminase in an adhesive.

In contrast, the present invention does not incorporate transglutaminase and is intended for injection into osseous defects or voids for local delivery of strontium and to aid in the formation of new bone. Literature data shows that elemental strontium is able to stimulate bone formation while simultaneously inhibiting bone resorption.

The present invention provides a composition that may be injected into an osseous defect or void or into the marrow space of trabecular bone tissue where it temporarily replaces bone marrow, to aid in the formation of new bone. The data shows that elemental strontium is able to stimulate bone formation while simultaneously inhibiting bone resorption.

The invention provides a composition for use in bone healing and bone growth wherein the composition comprises fibrin, thrombin and a strontium-containing compound. The composition may further comprise a calcium-containing compound. Preferably, the composition is an injectable composition that is in the form of an injectable gel, injectable putty, injectable paste or injectable liquid form.

In other embodiments, the composition may comprise one or more plasticizer agents. In certain embodiments, the plasticizer is an iodine-containing compound. Exemplary iodine-containing compound for use as plasticizers herein include but are not limited to compounds selected from the group consisting of diatrizoate, iodecol, iodixanol, iofratol, iogulamide, iohexol, iomeprol, iopamidol, iopromide, iotrol, ioversol, ioxagulate and metrizamide andmixtures thereof and polyvalent alcohols. In other embodiments, the plasticizer is a polyethylene glycol, a polyvalent alcohol, glycerol, or sugar selected from the group consisting of monosaccharides, disaccharides, trisaccharides, polysaccharides, and combinations thereof. In other embodiments, the placticizing effect is achieved by the adjustment of the composition of low molecular weight compounds such as, among others, sodium chloride and chaotropic agents.

The composition may be in a gel or a liquid form. In certain aspects, the composition can be delivered as a liquid or a gel prior to or during the gelling phase or as a pre-formed gel into tissue defects.

In other aspects of the invention the composition contains one or more extra-cellular protein selected from the group consisting of matrix proteins such as fibronectin, cellular associated proteins or plasma derived proteins such as blood clotting factor XIII and proteases.

In still other embodiments, the composition may further comprise a clotting inducing agent such as protamine, snake venom, transglutaminases, FXIIa, or a physiologically acceptable alkaline buffer system.

In specific embodiments, the composition is such that the composition is resorbed and replaced with tissue during the healing process.

The strontium containing compound used in the compositions of the invention is a 25% strontium substituted calcium salt.

The composition may be one which further comprises a strontium co-ligand selected from the group consisting of groups calcimimetics, osteocalcin, amino acids such as L- arginine, and, arginomimetics and arginine analogs.

In the compositions described herein, the strontium-containing compound and/or the calcium-containing compound may be in a (micro)particulate or solid form. In exemplary embodiments, the strontium-containing or calcium-containing (micro)particulate form has a particle diameter size ranging from 100 nanometers - 500 μm. In other embodiments, the strontium containing or calcium containing components can take the form of porous or non- porous solids with dimensions ranging from, for example 0.5 to 50 millimeters. Preferably, the particle size is in the range of 0.5 to 5 millimeters.

In specific embodiments, the calcium-containing compound is a calcium phosphate. The calcium phosphate may be selected from the group consisting of tricalcium phosphate, alpha-tricalcium phosphate, beta-tricalcium phosphate, polymorphs of calcium phosphate, hydroxyapatite, calcium carbonate, calcium sulfate and combinations thereof.

According to the present invention, some of the calcium is substituted with strontium to provide a mixture of calcium and strontium salts.

According to the present invention, the ratio or percentage of strontium salts to calcium salts is 25%. In certain embodiments, the compositions of the invention may further comprise a protein or combination of proteins selected from the group consisting of bone morphogenic proteins, parathyroid hormone (PTH), calcitonin, bisphosphonates, epidermal growth factor, insulin like growth factors and TGF growth factors. Any proteins that have a bone growth promoting, or bone augmenting effect may be used.

In certain other embodiments, the composition is one in which gelation of the composition occurs upon mixing of the various components.

In preferred embodiments, the methods using the composition of the invention comprise treating diseased, injured or deficient bone in a living subject by injecting into the cancellous bone of said subject a composition comprising a strontium-containing composition of the present invention. Advantageously, the injection of such a composition into the cancellous bone of the subject causes a rate of healing that is faster than observed with the application of a similar composition that does not contain strontium.

Also contemplated herein is a method of repairing a bone having a defect comprising: (a) mechanically fixing the bone or portions thereof; and (b) filling the defect with a composition of the invention in an amount effective to cause repair of said defect. In exemplary embodiments, the defect is a bone fracture and said repair is bone healing of said fracture. In specific aspects, the application of said composition causes a rate of healing that is faster than observed in the absence of application of said composition. Preferably, the application of said composition causes a rate of healing that is faster than observed with the application of a similar composition that does not contain strontium.

Also contemplated is a method of promoting new bone growth at the site of a bone defect comprising contacting said site of defect with a composition of the invention. Preferably the rate of said bone growth is faster in the presence of strontium in said composition as compared to bone growth seen in the presence of a similar composition that does not contain strontium.

Brief Description of the Drawings

Fig. 1 shows cell proliferation for human osteoblast-like cells (SaOS-2) grown on clots containing strontium. The notation used is Ca/Sr. The values quoted are the concentrations in the final clot. To achieve these concentrations in the final clot, the concentration in the buffer is doubled.

Fig. 2 shows a DNA assay for human osteoblast-like cells (SaOS-2) grown on clots containing strontium. The notation used is Ca/Sr. The values quoted are the concentrations in the buffer. This value is halved in the final clot.

Fig. 3 shows alkaline phosphatase production over 30 minutes for human osteoblast-like cells (SaOS-2) grown on clots containing strontium. The values quoted are the concentrations in the final clot. To achieve this the concentration in the buffer is doubled.

Fig. 4 shows turbidimetric analysis of clots for where the thrombin buffer is comprised of either a calcium, a calcium/strontium mix or strontium alone. The notation used is Ca/Sr. The values quoted are the concentrations in the buffer. This value is halved in the final clot.

Fig. 5 shows percentage survival (as measured by MTS proliferation assay) for clots grown in media containing 12.5mM Sr or Ca.

Fig. 6 shows staining of NHOst cells cultured on fibrin clots and fibrin clots containing strontium particles., Live (left panel) Dead (right panel) staining of NHOst cells cultured on fibrin clots for 14 days (Top panel) or fibrin clots containing strontium particles (Bottom panel).

Fig. 7 shows transverse sections of fibrin clots cultured with NHOst cells. Cells stain green (Live) and red (Dead). Top fibrin clot. Bottom fibrin clots containing particles.

Fig. 8 shows a graphical representation of completely closed drill holes (both sides are closed).

Fig. 9 shows the $\mu$CT of a rabbit femoral condyle in the presence of fibrin in the material.

Fig. 10 shows graphical representation of bone formation (evaluation: 1: low; 2: medium; 3: high bone formation).

Fig. 11 shows $\mu$CT of a rabbit femoral condyle with 80% Sr material.

Fig. 12 shows a histological stained thin section of a rabbit femoral condyle defect treated with Fibrin.

Fig. 13 shows a histological stained thin section of a rabbit femoral condyle defect treated with Sr 80% in a fibrin clot.

Fig. 14 shows the Influence of $SrCl_2$ on bioactivity of PTH.

The present invention is directed to a composition comprising fibrinogen, thrombin and a strontium-containing compound in an insoluble or soluble form and/or as an inorganic particulate, which may or may not contain a fibrin plasticizer for use in bone healing and bone growth. Alteration of the amounts of either the thrombin, or the inorganic particulate content, allows for control of the gelation period as the application or the target tissue dictate. A fibrin plasticizer or modifying agent can be used to enhance the mechanical properties of the formulation or to further delay the clotting time. Examples of plasticizers include iodine containing contrast agents such as diatrizoate, iodecol, iodixanol, iofratol, iogulamide, iohexol, iomeprol, iopamidol, iopromide, iotrol, ioversol, ioxagulate and metrizamide and mixtures thereof and polyvalent alcohols.

The composition can be delivered as a liquid prior to or during the gelling phase or as a pre-formed gel into tissue defects using a minimally invasive technique. It is intended that the formulation is resorbed and replaced with tissue during the healing process.

The fibrinogen solution is an aqueous solution with fibrinogen ranging from 5-200 mg/ml, preferably in the range 50-100 mg/ml. The fibrinogen solution can also include extra- cellular matrix proteins (e.g., fibronectin, cellular associated proteins, other plasma derived proteins [e.g., blood clotting factor XIII and proteases]. The fibrinogen solution can also include fibrin monomer, derivatives and fibrin I.

The thrombin component, may further comprise additional compounds known in the art as well as the strontium compound. There is no specific limitation in respect to the amount of thrombin used. In one example of the present invention, the amount of thrombin in said thrombin component (b) is such that it is at least about 1 IU/ml in the final clotted composition up to the amount of 30 IU/ml.

In place of or in addition to thrombin the present invention other gelation inducing or clotting inducing agent for component (a), such as protamine, snake venom, transglutaminases, FXIIa, a physiologically acceptable alkaline buffer system.

The thrombin buffer is an aqueous solution containing among other substances a divalent cation. The concentration of divalent cation in the buffer can range from 5-50 mM.

Preferably the divalent cations are chosen from the groups calcium and strontium and the ratio can be from 0:1 to 0.975:0.025 . The divalent strontium ions can be added in the form of strontium chloride or any other water soluble strontium salt such as strontium ranelate, strontium glutamate, strontium aspartate, strontium malonate, strontium maleate, strontium ascorbate, strontium threonate, strontium lactate, strontium pyruvate, strontium alpha-ketoglutarate or strontium succinate. The thrombin buffer can also contain co-ligands for a newly identified molecular target of strontium (a G-protein-coupled receptor calcium-sensing receptor, Pi et al., J. Biol. Chem. 2005). Co-ligands are selected from the groups calcimimetics, osteocalcin and amino acids. Preferably L-arginine, arginomimetics or arginine analogs. The strontium containing thrombin buffer can also be used in conjunction with the strontium containing inorganic particles.

The particulate component has a particle size diameter in the range of nano to less than 3000 $\mu$m. and should be insoluble at the time of application. Preferably, the particle size is between 100 nanometers to 50 millimeters and more preferably between 0.5mm to 50 mm. The particles can be strontium substituted calcium salts which are insoluble at the time of application. The calcium salt for substitution can be selected from the group consisting of calcium phosphates, tricalcium phosphates, alpha-tricalcium phosphate, beta-tricalcium phosphate, polymorphs of calcium phosphate, hydroxyapatite, calcium carbonate, calcium sulfate and combinations thereof. The strontium substitution of calcium of the particles is 25%. Mixing to obtain the final gel can be achieved over a range of temperatures 18-37 °C, preferably 37 °C.

The fibrinogen solution is an aqueous solution with fibrinogen ranging from 5-200 mg/ml, preferably in the range 50-100 mg/ml. The fibrinogen solution can also include fibrin monomer, derivatives and fibrin I. The fibrinogen solution can also include extra-cellular matrix proteins, e.g. fibronectin, cellular associated proteins, and other plasma derived proteins such as blood clotting factor XIII (FXIII) and proteases.

The thrombin solution is an aqueous solution with a minimum final concentration of 0.1 IU/ml in the final clotted (or gelled) composition. This solution can also be buffered and contain amino acids, proteins or additives ( for example L-arginine, mannitol and albumin).

The particle content in the composition ranges from 2-100 % preferably 30-50 % (weight/volume) of clottable proteins. The particulate component has a particle size diameter in the range of sub-micron to less than 3000 $\mu$m, as the application requires. Homogenisation to obtain the final gel can be achieved over a range of temperatures 18-37 °C, preferably 37°C.

The efficacy of the compositions of the present invention to aid in bone repair can be further improved by incorporating bioactive molecules. These can be chemically attached to the matrix, adsorbed on particulate component or trapped in the fibrin matrix either as a free molecule or as a drug powder. These molecules can be used to stimulate bone metabolism (anabolism and or catabolism). Suitable molecules that have been identified include bone morphogenic proteins (BMP), parathyroid hormone (PTH), calcitonin, bisphosphonates, epidermal growth factor, insulin like growth factors and the TGF super family growth factors. Suitable molecules can also include other factors not related to the bone anabolism/catabolism cycle but relating to physiological processes that occur in parallel to bone repair/ remodeling such as angiogenic stimulators (vascular endothelial growth factor (VEGF) and platelet derived growth factors [PDGFs] can also be incorporated. This is of particular interest as the mode of action of the strontium is believed to be separate from the anabolic agents 1,25(OH)2 vitamin D and parathyroid.

A plasticizer can also be mixed with the fibrin or thrombin to enhance the mechanical properties of the formulation or to further delay the clotting time. Examples of plasticizers are selected from the group consisting of water-soluble iodine containing contrast agents, polyethylene glycols, polyvalent alcohols such as glycerol, mono- , di-, tri- and polysaccharides, and any combination thereof. Suitable iodine containing contrast media are detailed in U.S. patent application number US 20050119746 (Lidgren and Nilson, 2004), examples of which are diatrizoate (meglumine), iodecol, iodixanol, iofratol, iogulamide, iohexol, iomeprol, iopamidol, iopromide, iotrol, ioversol, ioxaglate and metrizamide. Preferably the contrast agent is non-ionic, has a low osmolality and allows fibrin assembly to occur for example iodixanol.

The compositions of the present invention may be used in useful bone healing methods and in kits for use in such methods. It is contemplated that the composition may be used for promoting bone growth or healing especially in long bones. Specifically, the present invention relates to a bone repair composition that contains thrombin, fibrin and strontium. The presence of strontium in the composition leads to enhanced bone healing and or bone generation as compared to such a composition that does not contain strontium.

The compositions of the invention may be used in repairing and regenerating long bones such as the femur, tibia, humerus, radius ulna and the like.

Using the compositions of the present invention it will be possible to restore or partially restore mechanical, architectural and structural competence to bones having defects, while providing structural surface areas, which can serve as appropriate substrates for the biological process governing bone healing and regeneration.

As shown in the examples, the composition of the invention accelerates the cellular and biological processes involved in bone repair.

As used hereinafter the phrase "long bone" refers to a bone having a length, which is at least two times longer than the diameter. Typically, the phrase "long bone" refers to the bones of the extremities, i.e. the tibia, fibula, femur, humerus, radius, ulna, carpals, metacarpals, phalanges, tarsals and metatarsals. More specifically, the phrase "long bone", as used herein, refers to the four main bones of extremities, i.e., the tibia, the femur, the humerus and the radius.

The "bone defect" that may be remedied by use of the compositions of the present invention may be any abnormality in the bone, including, but not limited to, a void, a cavity, a conformational discontinuity, a fracture or any structural change produced by injury, osteotomy, surgery, fractures, malformed healing, non-union fractures, skeletal deformations, aging, or disease. The compositions of the present invention provide significant therapeutic potential. When cancellous bone becomes diseased, for example, because of osteoporosis, avascular necrosis, or cancer, the surrounding cortical bone becomes more prone to compression fracture or collapse. This is because the cancellous bone no longer provides interior support for the surrounding cortical bone. The compositions of the invention can be used in strengthening bone in conditions such as osteoporosis, osteonecrosis or avascular necrosis as well as other bone disease involving infected bone, poorly healing bone, or bone fractured by severe trauma.

Described herein is a kit for repairing a bone having a defect. The kit includes a mechanical fixing device for fixing the bone or portions thereof and compositions of the present invention. Examples of a fixing device include, but are not limited to, flanges, rods, bars, wires, staples, screws, sutures as well as various casts, sleeves and the like, which are typically external fixing devices. Hence, the kit described herein preferably includes an external mechanical fixing device.

Described herein is a method of treating bone defects, such as fractures, cavities voids and the like comprising contact them with a composition of the present invention. In some embodiments, the method may involve mechanically fixing the bone or portions thereof prior to, or following a step of contacting the defect with a composition of the present invention.

Advantageously, the compositions of the present invention may be used as injectable compositions that are delivered into an osseous (bone) defect or void or into the marrow space of trabecular bone tissue where the composition temporarily replaces bone marrow, and/or aids in the formation of new bone. The compositions may be injected into trabecular bone tissue or any other bone tissue using techniques well known to those of skill in the art. Exemplary such techniques and apparati for performing such techniques are discussed in for example, U.S. Patent publication No. 20080065091, U.S. Patent publication No. 20080058828, U.S. Patent publication No. 20070073307, U.S. Patent Nos. 4,969,888 and 5,108,404 among others. Typically, injection devices similar to a household caulking gun are used to inject the compositions into bone. A typical bone cement injection device has a pistol shaped body, which supports a cartridge containing bone cement. A trigger actuates a spring-loaded ram, which forces a volume of bone cement in a viscous condition through a suitable nozzle and into the interior of a bone targeted for treatment. According to the teachings of U.S. Patent. Nos. 4,969,888 and 5,108,404, a cavity can be first formed by compacting cancellous bone inside the bone, into which the bone cement is injected.

An added advantage of incorporation of the Sr into the injectable compositions of the invention is that it allows the delivery and healing process to be monitored using a technique such as fluoroscopy due to the known properties of strontium as a radio-opacifier. As such, the use of the Sr in the compositions of the present invention provides both a therapeutic and diagnostic function and not simply an imaging function alone.

The compositions of the present invention may be used in the form of an injectable gel, an injectable paste, a paste, a putty, or a rehydratable freeze-dried form. As used herein, the term term "gel" refers to a jelly-like, thick, soft, partly liquid substance. A gel of the present invention may be extruded through at least a 13 gauge syringe needle. "Paste," as used in the present application refers to a soft, moist, substance having a consistency between a liquid and a solid. A paste of the present invention is less solid than a putty and more solid that a gel, and in some embodiments may be injectable.

The term "putty" refers to a dough-like/clay-like tissue repair composition of the present invention. During application the substance may be beaten or kneaded to the consistency of dough, and molded into a shape closely approximating that of the implant site.

"Injectable" refers to the ability of the present invention to be introduced at an implant site under pressure (as by introduction using a syringe). An injectable composition of the present invention may, for example, be introduced between elements or into a confined space in vivo (i.e., between pieces of bone or into the interface between a prosthetic device and bone, among others).

"Syringe" refers to any device that may be used to inject or withdraw flowable tissue repair compositions of the present invention, including certain gels and pastes, among others.

The composition is injected into cortical bone or trabecular bone of a subject (preferably, the subject is a human, simian, ovine, bovine, equine, porcine subject). "cortical bone," as used in the present application, refers to the compact bone of the shaft of a bone that surrounds the medullary cavity. Cortical bone is a highly dense structure made up of triple helix strands of collagen fiber, reinforced with hydroxyapatite. The cortical bone is a compound structure and is the primary load bearing component of long bones in the human body. The hydroxyapatite component is responsible for the high compressive strength of the bone while the collagen fiber component contributes in part to torsional and tensile strength. The compositions of the invention may be introduced into such bone parts to impart a beneficial bone healing or bone augmentation effect.

Trabecular bone is of similar composition to cortical bone and is the primary structural component of "cancellous bone" and refers to adult bone consisting of mineralized regularly ordered parallel collagen fibers organized differently than in the lamellar bone of the shaft of adult long bones. Cancellous bone is generally found in the end of long bones surrounded by cortical bone. Cancellous bone has spicules that form a latticework, with interstices filled with bone marrow. It may also be referred to as a trabecular bone, or spongy bone. In preferred embodiments, the compositions of the invention are delivered into trabecular bone.

The bone-fixing methods described herein also may include the step of reshaping the defect prior to bone fixing or contacting with the composition of the invention. Such reshaping or bone realigning can be performed with, for example, drills or grinders or any other device utilizable for reshaping the defect.

The device used for fixing the bone will depend on the type of defect to be repaired and on the type and placement of the bone. Typically, the fixing device is an external fixing device. Preferably, an external fixing device such as, for example, a cast, is the only fixing device utilized in the method described herein, accompanied with an injection of the composition of the present invention. However, in some circumstances it may be necessary to employ more invasive surgical procedures using an internal fixing device can be utilized as well.

Repair can be monitored and in due course the device employed to fix the bone may be removed. Bone regeneration at the site of the defect can be assessed by soft tissue X-rays (7.5 mA; 0.5 sec) taken immediately following surgery and at given time intervals post surgery. Bone regeneration in test animals also can be determined upon termination of the experiments by assessing general morphology, computerized topography (CT) scan (65-80 kV; 20 sec) and three-dimensional (3-D) CT scan (Marconi, M.times.8,000). Histological studies can be performed to determine the bone staining of the tissue.

In the methods described herein, the compositions also may be combined with cells that can be used in bone repair, such as for example, osteoprogenitor cells. Osteoprogenitor cells, as is known in the art, include an osteogenic subpopulation of the marrow stromal cells, characterized as bone forming cells. The osteoprogenitor cells utilized by the method of the present invention can include osteogenic bone forming cells per se and/or embryonic stem cells that form osteoprogenitor cells. The osteoprogenitor cells can be isolated using known procedures. Such cells are preferably of an autological source and include, for example, human embryonic stem cells, murine or human osteoprogenitor cells, murine or human osteoprogenitor marrow-derived cells, murine or human osteoprogenitor embryonic-derived cells and murine or human embryonic cells. These cells can further serve as cells secreting growth factors, as described by Robinson and Nevo (2001), which are defined hereinabove.

The composition of the present invention also may further comprise various active therapeutic agents, which can include bone growth-promoting agents, osteoprogenitor cells or a combination thereof. The compositions may include at least one drug, such as, a vitamin, an antibiotic, an anti-inflammatory agent and the like.

According to the present invention, the strontium is incorporated into a calcium containing salt using techniques known to those of skill in the art to prepare strontium substituted calcium compounds.

A "bioactive glass" is any glass that displays characteristics of bioactivity, it is typically and an amorphous solid that is not intrinsically adhesive and that is capable of forming a cohesive bond with both hard and soft tissue when exposed to appropriate in vivo and in vitro environments, such as simulated body fluid or tris-hydroxymethylaminomethane buffers. A cohesive bond is achieved by developing a surface layer of hydroxycarbonate apatite onto the bioactive glass through the release of ionic species from the bulk bioglass material. There are numerous applications for bioactive glasses in the field of surgical and orthopedic treatments as well as in dental surgery and have been described, for example, in European Patent 1 405 647 and European Patent Application 1 655 042, as well as in International applications WO 96/21628, WO 91/17777, WO 91/12032 and U.S. Patent publication number 20080066495.

Examples:

In one embodiment described herein, the strontium-containing compound is used as the divalent ion in a thrombin dilution buffer. The thrombin solution is prepared using the dilution buffer. The fibrinogen solution and the strontium containing thrombin solution are mixed to form a gel.

According to the present invention, the strontium-containing compound is added to the formulation as a salt particle. The thrombin solution and particles are mixed to prepare the modified thrombin solution. The fibrinogen solution and the modified thrombin solution are mixed to form a gel.

**Example I: Strontium Containing Compound Dissolved in the Buffer Solution.**

A series of buffers were prepared in double distilled water. For these buffers, the divalent cation in the thrombin buffer was Ca or a Ca + Sr mixture such that the cation concentration was kept constant at 40 mM (see table below for strontium chloride). The thrombin was diluted to a concentration of 4IU in the thrombin buffer.

| $CaCl_2$ 40mM | $CaCl_2$ 30mM | $CaCl_2$ 20mM | $CaCl_2$ 10mM | $CaCl_2$ 0mM |
|---|---|---|---|---|
| $SrCl_2$ 0mM | $SrCl_2$ 10mM | $SrCl_2$ 20mM | $SrCl_2$ 30mM | $SrCl_2$ 40mM |

The fibrinogen was then mixed with thrombin in a 1:1 ratio (therefore the strontium concentration in the gelled clot is halved). For this, 2 ml of the thrombin solution can be transferred to a 5ml syringe. 2 ml of fibrinogen (Tisseel, Baxter, Clottable Protein, [fibrinogen and fibronectin] 72-110 mg/ml) was transferred to a separate 5ml syringe. The syringes containing the fibrinogen and the thrombin can be connected to any state of the art mixer for the purpose of combining.

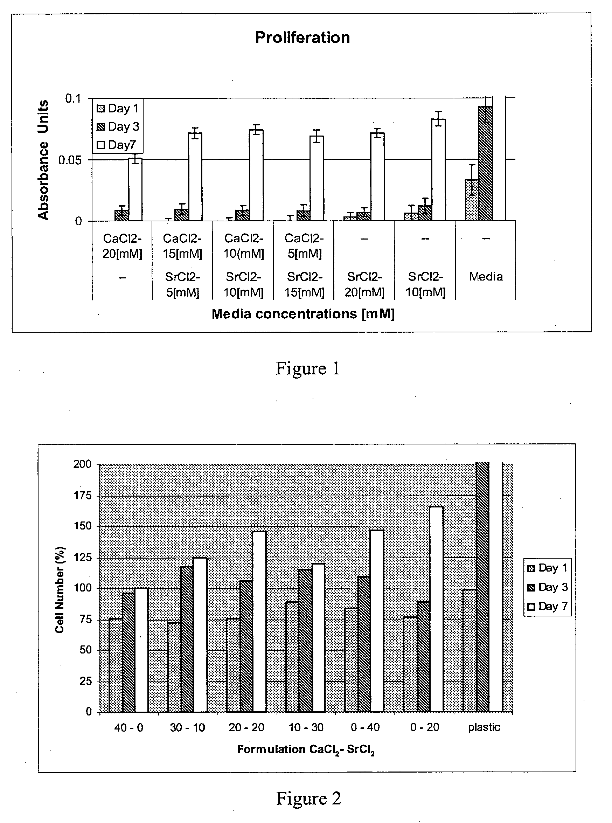

For *in-vitro* experiments, the clots were prepared by pipetting 150 μl of the fibrinogen solution to the wells of a 24 well plate. The plate was put on plate shaker to ensure an even distribution over the well bottom. 150 μl of the thrombin solution was then added to the well and the plate was put on a plate shaker to ensure homogeneous clots. A human osteoblast-like cell line (SaoS-2) was cultured on the clots for up to 7 days to see if any differences in cell proliferation/cell differentiation could be observed. Figure 1 shows the results for proliferation (Alamar Blue) when Strontium Chloride (SrC12) is added to the thrombin dilution buffer. Similar results are available for Strontium Acetate (SrAc).

The Alamar Blue assay is a metabolic assay and is often used to measure proliferation. The addition of strontium resulted in significant increased proliferation on these clots when compared to a normal clot with 20mM $CaCl_2$. A similar result was also observed when the proliferation was quantified using a DNA stain (Figure 2). After 7days, clear differences could be observed between cells grown on clots containing either a calcium strontium mix or only calcium. Thus we conclude that strontium has a positive effect on the proliferation of osteoblast like SaOS-2 cells.

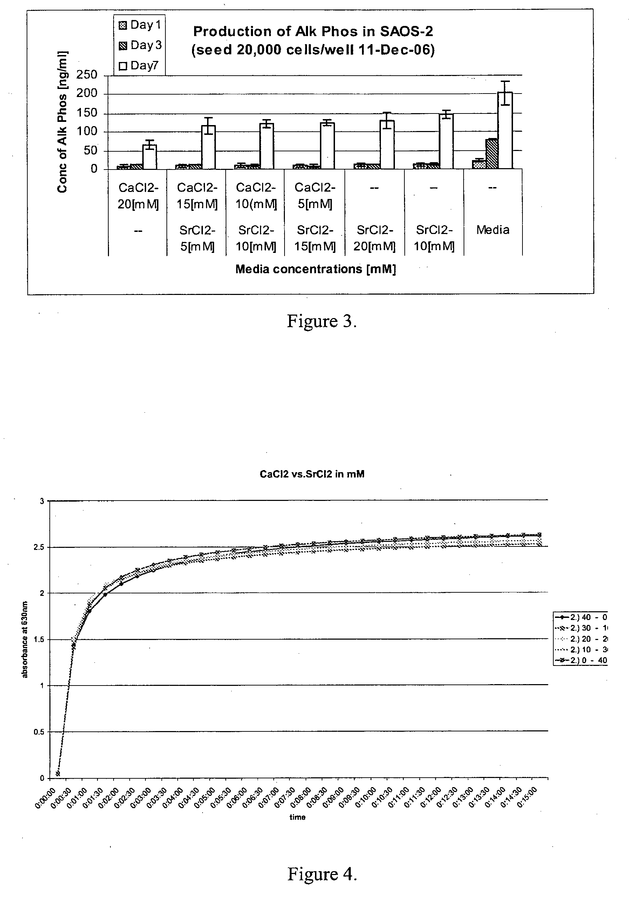

These initial experiments were designed to look at differences in cell proliferation did not provide sufficient time to investigate osteoblast differentiation. The enzyme alkaline phosphatase is an early indicator of differentiation but levels do not really peak till about days 10-14. A preliminary result shows an increase in alkaline phosphatase expression in the clots containing strontium (Fig. 3). In the case of strontium acetate these values appear not to be significant. These experiments are being repeated with a 14 day investigative period.

In addition to the cell studies observation, turbidmetric analysis was carried out to examine if the clot structure was altered as a result of the strontium (Figure 4) The clot turbidity is directly proportional to average cross-sectional area of the fibres, therefore the more turbid the clot the greater the fiber diameter.

The turbidimetry data in Fig. 4 shows that there is very little difference in the kinetics and absorbance for strontium containing clots in comparison to calcium containing clots. This may be explained by observations that calcium and strontium are capable of occupying similar binding sites, in particular the FXIII binding site.

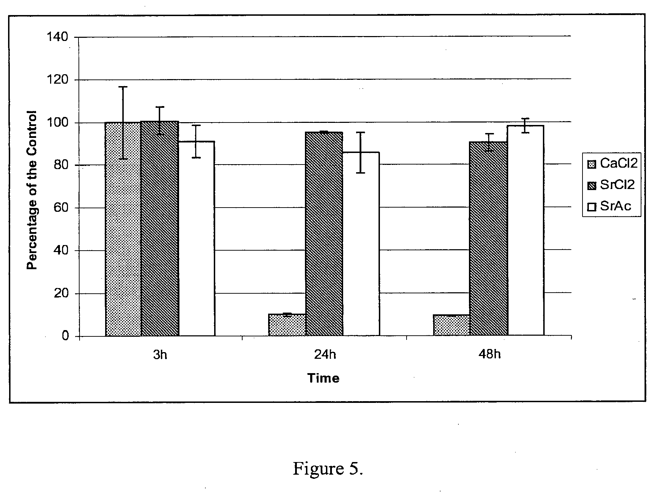

For the initial experiments, cells were seeded on the surface of clots. One of the reasons for choosing this seeding method is that some cell types when seeded within the clots suffer largely from cell death and there is migration of the cells to the surface of the clot. One possible explanation for this effect may be explained when osteoblast-like cells are cultured in a medium supplemented with $CaCl_2$. Figure 5 shows the result of SaOS-2 cell culture on tissue culture plastic in the presence of 12.5 mM $CaCl_2$. Cell death (as measured by an MTS assay) occurs for the cells cultured in the presence of the calcium chloride. This occurs within the first 24 hours of the cell culture. By contrast there is only a slight reduction in the proliferation of cells cultured in the presence of strontium containing compounds. It is important to point out that some cells have a requirement for high calcium i.e., fibroblasts and keratinocytes, and thus would not affected by the calcium concentration of the clot.

Example 2: Fibrin/Strontium Doped-Calcium Phosphate nanoparticles.

Thrombin was diluted to a concentration of 4 IU in the thrombin buffer. The fibrinogen was mixed with thrombin in a 1:1 ratio. For this 2 ml of the thrombin solution can be transferred to a 5ml syringe. 2 ml of fibrinogen (Tisseel, Baxter, Clottable Protein, [fibrinogen and fibronectin] 72-110 mg/ml) was transferred to a separate 5ml syringe. The particles (nanoparticles less than 1 $\mu$m) are incorporated as a percentage weight of the final clot volume ((w/v). These are weighed and placed into another 5ml syringe.

The syringes containing the particles and the thrombin are connected via a Luer adapter and the thrombin and particles homogenised by transferring the contents from syringe to syringe. The syringes containing the thrombin/particles and the fibrinogen are connected via a Luer adapter and the contents homogenised. The material remains liquid for approx 30 seconds during this time it can be injected into the defect.

For *in-vitro* experiments, the clots are prepared by pipetting 150 $\mu$l of the fibrinogen solution to the wells of a 24 well plate. The particles (nanoparticles less than 1 $\mu$m) are added as percentage weight of the final clot volume (w/v). The plate is put on plate shaker to ensure an even distribution over the well bottom. 150 $\mu$l of the thrombin solution is then added to the well and the plate put on a plate shaker to ensure homogeneous clots. In the preliminary studies, a human osteoblast like cells (SaOS-2 or NHOst) were cultured on the clots for up to 14 days to see if any differences in cell proliferation, cell differentiation could be observed.

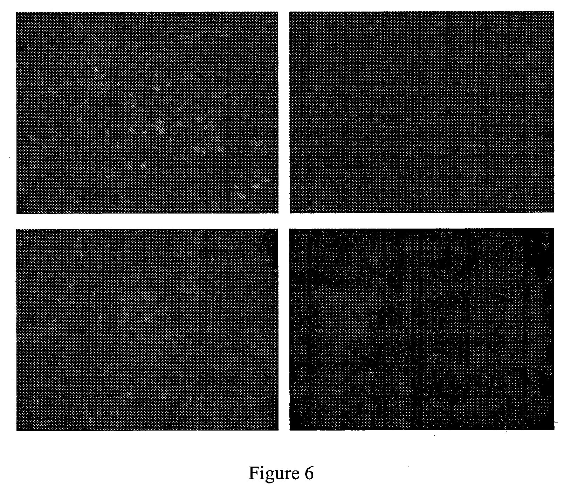

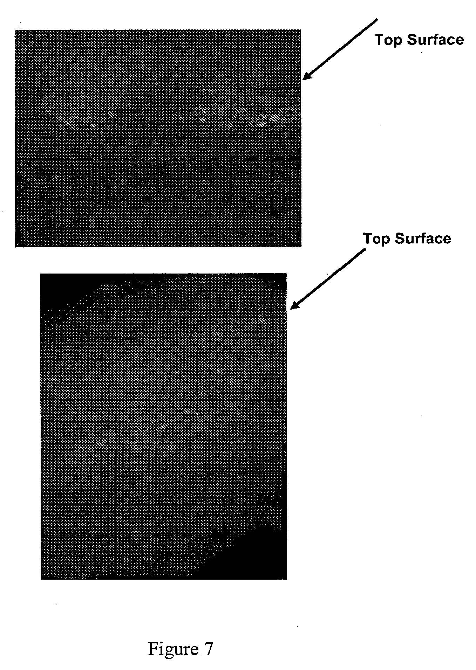

Qualitative analysis for cells seeded on the particle containing clots show good biocompatibility, when compared to normal fibrin clots (Figure 6). Although not quantified, it appears that the cells on the fibrin clots without particles are more rounded in morphology while those on the particle containing clots are more spread. Further evidence of this can be seen in transverse sections of the clots (Figure 7). For this, cells were seeded in the clots. After a few days the cells in the fibrin clots had migrated to the surface of the clot. By comparison, cells in the particles containing clots remained in the clots and have spread out showing a favorable morphology.

Example 3: Use of Strontium Doped Hydroxyapatite In Rabbit Femoral Confyle Defect Model.

The studies described above suggest that strontium stimulates bone formation and at the same time represses bone resorption. Previous studies investigated the effect of SrC12 on osteoblast like cells and on primary osteoblasts. In the course of the attempt to identify a formulation that can be used in vertebral augmentation, a modified hydroxyl apatite that was doped with strontium, was tested in a rabbit femoral condyle defect model to assess the potential of strontium to stimulate bone formation. Calcium hydroxyl apatite can be produced at the bench by stirring calcium nitrate and ammonium phosphate together at a high pH value (adjusted with ammonium hydroxide). The resulting precipitate can be centrifuged, washed, dried and calcined to obtain a hydroxyl apatite like substance. By combining calcium nitrate with strontium nitrate in the respective percentage of substitution it is possible to produce strontium-calcium hydroxyl apatite like particles that can be injected together with fibrin into a bone defect.

This example shows data that shows that strontium substituted calcium hydroxyl apatite like particles stimulate new bone formation better than fibrin alone or pure calcium hydroxyl apatite like particles. These results provide *in vivo* corroboration of the data obtained during preliminary cell culture.

**Materials and Methods:**

The following chemical reagents were used in the studies described in the present example. Calcium nitrate tetrahydrate; 99% A.C.S.reagent [Sigma-Aldrich; 237124-500G; FW: 236,15]; Strontium nitrate p.A. >99% [Fluka; 85899 500g; Lot&Filling Code: 1086321, 53706181; FW: 211,63]; Ammonium phosphate biphasic p.A. [Riedel-de Haën; 30402 500g; FW: 132,06] ; Ammonium hydroxide [Sigma-Aldrich, 318612-2L; batch#: 11103PD; FW: 35,05; 5N in $H_2O$]; EtOh 96%; Fibrinkleber [Baxter AG; Fibrinkleber TIM 3; 08P6004H; 5ml; 1500434]; Thrombin [Baxter AG; B205553 thromb. SD TIM 5; 500IE; US 5ml]; Calcium chloride dihydrate (min. 99%) [Sigma; C7902-1KG; batch#: 044K0160]; and Sodium chloride [Merck, 1.06404.1000 1kg; Charge/Lot: K38062004 745; FW: 58,44.

The experiments employed various solutions as follows: 40mM $CaCl_2$+200mM NaCl: 1.47g $CaCl_2$ + 2.92g NaCl

in 250ml ddH$_2$O; lyophilized thrombin- reconstituted in 5ml 40mM CaCl$_2$+200mM NaCl and lyophilized fibrinogen - reconstituted in 5ml of 3000 KIE/ml Aprotinin.

Experiments were performed on 4 tests groups with 6 rabbit knees each as follows:

Group 1. fibrin + thrombin (8IU/ml final concentration)
Group 2. fibrin + thrombin (8IU/ml final concentration) + 100% Ca (0% Sr substitution)
Group 3. fibrin + thrombin (8IU/ml final concentration) + 75% Ca (25% Sr substitution)
Group 4. fibrin + thrombin (8IU/ml final concentration) + 20% Ca (80% Sr substitution).

**Preparation of Test Materials:**

Fibrinogen was reconstituted and aliquoted in 1ml syringes [Braun; omifix - 1ml; Luer] with 0.5ml fibrinogen per syringe. Thrombin was reconstituted and diluted with 40mM CaCl$_2$+200mM NaCl to 16 IU/ml. This was also aliquoted in 1ml syringes, 0.5ml per syringe.

The hydroxyl apatite powders were produced by stirring together equal volumes of 2M calcium nitrate solution (or according to percentage of strontium substitution also strontium nitrate, the resulting solution has to have 2M in total) and 1.2M ammonium phosphate solution. Upon addition of ammonium phosphate to the calcium nitrate a pasty substance precipitated. A subsequent addition of an equal volume of ammonium hydroxide elevated the pH of the precipitate to 11 and made the pasty precipitate liquid. After thorough stirring the mixture was centrifuged, the supernatant was discarded and the precipitate was washed two times with alcohol (ethanol 96%) and one time with ddH$_2$O. Between the washing steps the precipitate was always elutriated, centrifuged and the supernatant was discarded. Thereafter the precipitate was dried at 60°C under vacuum for one day, milled and calcined for 1 hour at 1100°C.

After cooling the hydroxyl apatite like particles were sterilized with heat and aliquoted in 1ml syringes, 0.3g per syringe.

**Treatment of Rabbits**

12 rabbits were used to perform the main experiment. They were sedated and a bore hole (diameter 4.5mm) was drilled into the femoral condyles. These drill holes were filled each with one of the test materials by swooshing the thrombin component with particles and thereafter with fibrinogen, or just thrombin with fibrinogen as a control. The cone of the 1ml syringes fit perfectly into the drill holes, so a direct application was possible.

After injection of the respective material (randomized for the 24 knees) the skin was sewed and the rabbit was kept monitored for the following 8 weeks.

**Post-operation Analysis**

After the 8 weeks the rabbits became euthanized and the knees were analysed by $\mu$CT and thin sections were histologically stained.

**Results**

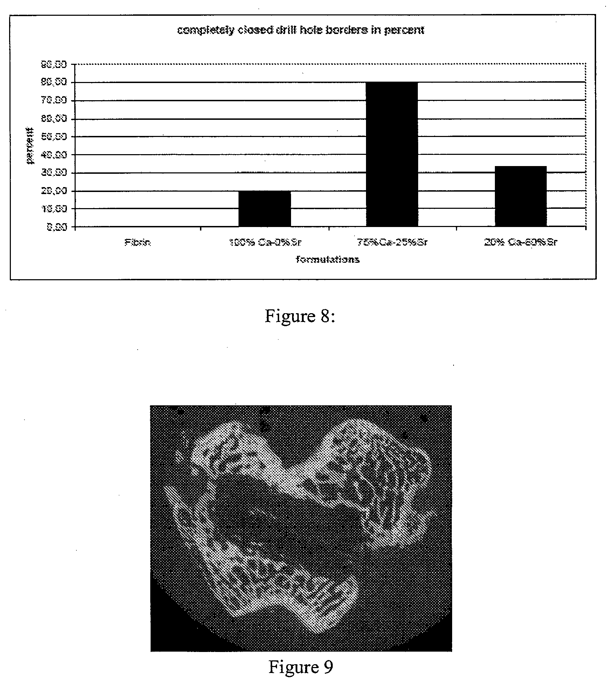

$\mu$CT analysis: Due to the high opacity of the strontium samples it was difficult to obtain quantitative results, so the analysis was done semi-quantitative by assessing the $\mu$CT pictures with the eye and quantifying them. Evaluation of bone formation was performed in three levels (1: low; 2: medium; 3: high). The evaluation of a closed drill hole was done by giving a "+" for a closed drill hole and a "-" for a not closed drill hole. The data are shown in graphs at Figures 8 through 14. All graphs are averages of evaluable treated animals, whereas the $\mu$CT pictures are snap shots.

Figure 8 shows graphical representation of completely closed drill holes (both sides are closed). None of the evaluated rabbit condyle defects filled with fibrin was closed on both sides, hence zero percent completely closed drill hole borders are seen in Figure 8. The substitution of 25% Sr in the particles yielded the highest value of 80% closed drill hole borders.

In Figure 9, there is the $\mu$CT of a rabbit femoral condyle in the presence of fibrin. The defect is still visible, but no residual material (fibrin) is visible; one side of the drill hole is still open.

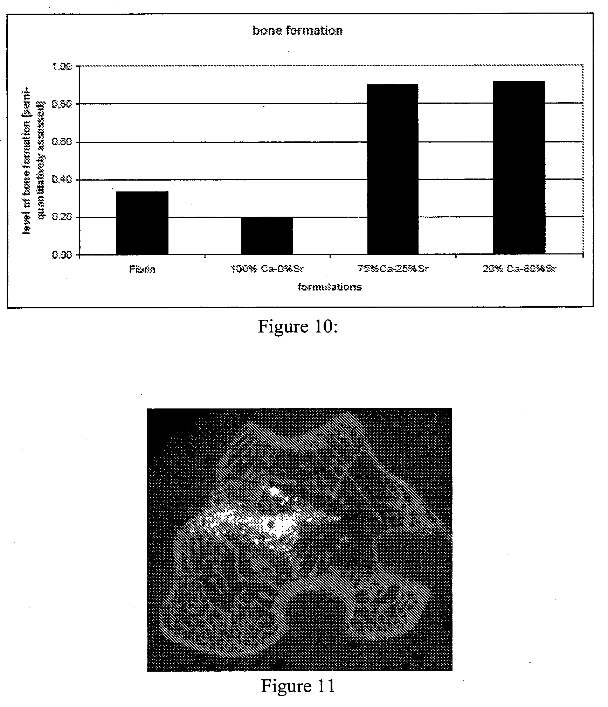

Addition of strontium produces marked effects. Figure 11 shows $\mu$CT of a rabbit femoral condyle in 80% Sr material. While the defect is still visible, most of the defect is filled; the material (Sr80%) is strongly visible. New bone formation is clearly identifiable and both sides of the drill hole are closed.

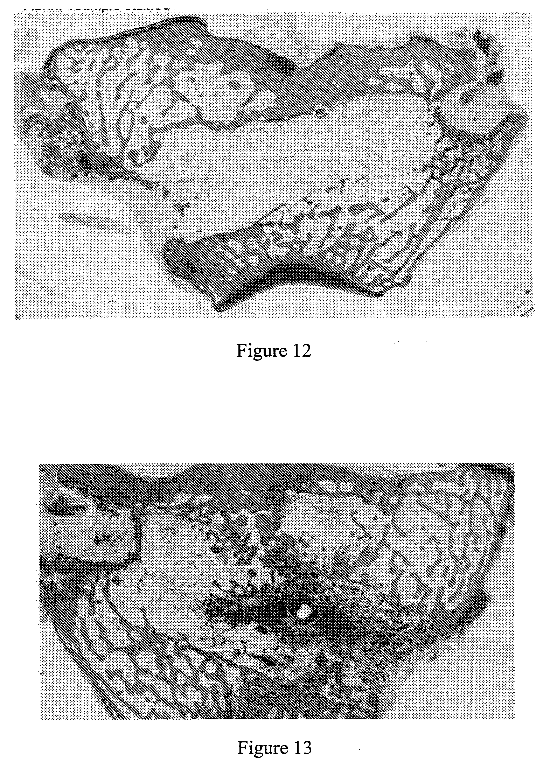

The above results also were confirmed using histological studies. Figure 12 shows a histological stained thin section of a rabbit femoral condyle defect treated with fibrin. No test substance is present. Accordingly no signs of inflammation are found. Only a few, short cancellous bone trabeculae are found inside the drill hole. At the opening of the bur canal, the newly formed bone substance is of cancellous shape. Blood vessels are almost regularly distributed inside the drill cavity, showing only few spots that are less vascularised. Several larger blood vessels are in close vicinity to the bone substance at one side of the drill cavity.

In the samples treated with 80% strontium in the fibrin clot (Figure 13), 40-70% of the drill canal is filled with the test substance. The test substance is in all samples of elongated shape inside the drill cavity. In one sample it is more granular, while the other two exhibit a coarser test substance. In 2 of 3 samples fibrin is present as large patches close to the test substance. Inflammations are few in one sample, and vary from 3-10 large inflammations in the other specimens. In one sample the test substance is in direct contact to the newly built bone tissue, whereas it lies more distantly in the other two. Two samples have regularly distributed blood vessels inside the drill cavity, while the third has a more clustered vascularisation.

**Discussion**

The $\mu$CT data shows that formulations containing Sr 25% substituted hydroxyl apatite like particles trigger a closure of a drill hole better than the other tested substances, induce a comparable bone formation to 80% substituted particles, while being less dense.

Fibrin alone triggers less bone formation and the drill holes are closed only on one side. Formulations containing pure calcium hydroxyl apatite like particles are not so dense, but seem not to induce bone formation or a closure of the drill holes.

The high strontium concentration formulation is very dense, it induces new bone formation, but not significantly more than Sr 25%. Furthermore drill hole closure is worse compared to Strontium in a lower concentration, thus even low concentrations of strontium is sufficient to induce bone formation.

Histological results suggest that the substitution of strontium in calcium hydroxyl apatite like particles induces formation of new bone. Vascularisation, a prerequisite for the immigration of osteoblasts and hence new bone formation, was detected in all the samples.

Example 4: Strontium has a synergistic effect with PTH on osteoblast cells.

The present example describes *in vitro* studies on rat osteosarcoma cells to investigate if a combination of parathyroid hormone (PTH) and Strontium (used as SrCl2) on activation of bone formation factors like increased cAMP production in osteoblasts. The studies described herein show that SrC12 has a positive synergistic effect with PTH in generation of cAMP in osteoblast cells. No such effect was observed for cells treated with $CaCl_2$. As cAMP is known to induce anabolic bone formation, the data presented herein support a conclusion that a combination therapy of Sr and PTH will lead to increased bone formation in vivo.

**Materials and Methods**

The osteosarcoma cell line UMR-106 (NewLab Bioquality AG, Erkrath, Germany) was used to perform the bioassay.

The assays described below used the following media:

*Cell culture medium:* DMEM (Sigma, D6546; incl. 4500mg/l Glucose, Na-Pyruvate, Na2CO3, suppl. with 0.584g/l L-glutamine); 10% FCS; 2mM L-Glu.
*Starvation medium:* DMEM (Sigma, D6546; incl. 4500mg/l Glucose, Na-Pyruvate, Na2CO3, suppl. with 0.584g/l L-glutamine); 2mM L-Glu;
*SI medium:* 10 ml starvation medium; 2 mM IBMX
*20 mM SrCl2 in starvation medium:* 19,6 ml Medium 0,4 ml 1M $SrCl_2$
*20 mM CaCl2 in starvation medium* 19,6 ml Medium 0,4 ml 1M $CaCl_2$.

**Cell Culture**

UMR-106 cells were seeded in growth medium at a density of 30000 cells/cm$^2$ and incubated at 37°C/ 8.0% $CO_2$ After 24 hours, medium was exchanged with either fresh medium, medium containing 20 mM $SrCl_2$ or medium containing 20 mM $CaCl_2$. Cells were further incubated at 37°C/ 8.0% $CO_2$ for 24 hours. Cells were harvested according to the following procedure:

Cells were washed twice with HBSS and detached from the surface using 6ml trypsine/EDTA for 3min at room temperature. Trypsin digestion was stopped with 12ml growth medium. Following centrifugation (3min, 1000rpm, RT) cells were resuspended in 12ml starvation medium and counted with a CASY cell counter. The rate of dead cells was below 10% in every experiment. Cells were resuspended to a final concentration of $1.6 \times 10^6$ cells/ml in starvation medium. 50$\mu$l of cell suspension was transferred into each well of a 96-well plate and incubated for 30min at 37°C, 8.0%$CO_2$.

## Treatment of cells with 264 $\mu$g/ml TGpIPTH samples in fibrinogen solution

Samples were diluted in freshly prepared starvation medium containing 2mM IBMX (SI medium). For preparation of SI-medium 30 $\mu$l of a 0.67M IBMX stock in DMSO was added to 10 ml of starvation medium.

All sample dilutions were prepared with this medium to inhibit phosphodiesterase activity in UMR-106 cells (Janik, P., 1980; Chasin M. and Harris, D.N., 1976). 50$\mu$l of diluted samples (as indicated in results) were added to the cells after 30min incubation at 37°C. For cAMP production cells were incubated for 1h at 37°C, 8.0%$CO_2$. Each sample concentration was added to cells at least in duplicates following cAMP analysis in duplicates.

## cAMP Biotrak Enzyme Immune Assay

*Reagent preparation for cAMP EIA.* Assay buffer, lysis reagent 1A/1B, lysis reagent 2A/2B, cAMP standard, antiserum, cAMP Peroxidase conjugate and wash buffer were prepared according to the manufacturer's manual (cAMP Biotrak EIA kit, GE Healthcare). 1M sulfuric acid stop solution was prepared by dilution of 53ml $H_2SO_4$ (conc.= 18.76M) with 947ml dd$H_2O$.

*Cell lysis and dilution of CAMP* For extraction of cAMP 100$\mu$l of cell suspension containing PTH was incubated with 25$\mu$l (final dilution 1:5) lysis reagent solvent 1A that is part of the cAMP Biotrak EIA kit (GE Healthcare). For total lysis, cells were shaken for 15min at 500rpm at room temperature. The extracted cAMP was finally diluted 1:20 with lysis reagent 1B (cAMP Biotrak EIA kit) (1:1 dilution in the assay plate (125$\mu$l lysis reagent 1B were added) followed by 1:10 dilution on the ELISA plate (90$\mu$l lysis reagent 1B + 10$\mu$l diluted cell suspension).

*Preparation of cAMP working standard.* The lyophilized cAMP standard for non-acetylated assay (cAMP Biotrak EIA kit) was resolved in assay buffer to get a concentration of 32pmol/ml. A double dilution series was prepared ranging from 32pmol/ml to 0.5pmol/ml.

*Internal control.* 100$\mu$l of each standard and sample dilution were transferred to the appropriate well and analyzed in duplicates. Additionally, two different internal controls, the non-specific binding (NSB) control and a zero control (0) were necessary to show the specificity of the cAMP ELISA and were performed as recommended by the manufacturer (GE Healthcare).

*Enzyme Immunossay procedure.* 100$\mu$l of antiserum was added to all wells except the NSB control. Antibody reaction was performed for 2h at 4°C with shaking. Following incubation wi th 50$\mu$l cAMP peroxidase conjugate for 1h at 4°C with shaking, all wells were washed four times with 300$\mu$l wash buffer. Finally, 150$\mu$l TMB (3,3',5,5'-Tetramethylbenzidine) was added to each well and substrate reaction was allowed for 10min at room temperature. The reaction was stopped by adding 100$\mu$l 1M $H_2SO_4$ and the optical density was determined immediately in a plate reader at 450nm.

*Data analysis.* The optical density was determined in a plate reader (Synergy HT) by measuring the absorbance of the samples at 450nm using the software KC4. Background corrections, calculations of mean values, standard deviations, coefficients of variation and generation of standard curves were performed in Microsoft Excel 2000. Sigmaplot 9.0 was used for 4-parameter-fits and EC50 calculations. PLA1.2 (Stegmann Systemberatung) was used for Parallel Line Analysis and detection of Relative Potency.

*Percentage of bound cAMP peroxidase conjugate.* The percent bound cAMP peroxidase conjugate for each standard and sample was calculated using the following relationship:

$$B/B0\ (\%) = \frac{(ODsample - ODnsb) \times 100}{(ODzero - ODnsb)}$$

OD sample......OD450 of specific sample
OD zero...........OD450 of peroxidase conjugated cAMP only
OD nsb............OD450 without cAMP antibody

*Standard curve.* The cAMP standard curve was generated by plotting the percent B/B0 (y-axis) as a function of the log c(cAMP) (x-axis).

*cAMP concentration of the samples.* Taking into account the dilution factor (20x) the produced cAMP amounts were calculated using the parameters (slope and intercept) of the standard curve.

*Linearity.* The correlation coefficient of standard curve of the cAMP ELISA ($R^2 \geq 0.95$) was evaluated

*Preparation of Samples for Bioassay Analysis.* For analysis, samples were diluted in starvation medium containing 2mM phoshodiesterase inhibitor IBMX (SI-medium) to a final concentration of 2400nM TGplPTH (13,2 $\mu$g/ml TGplPTH). For all samples 150$_\Lambda$1 of the following 8 serial dilutions were prepared and 50$\mu$l were added directly to the cells (total volume on cells: 100$\mu$l). For all samples and all dilutions, mean values of OD450, standard deviations, cAMP concentrations and coefficients of variations were calculated.

| c(TGplPTH) nM | preparation | c(TGplPTH) $\mu$g/ml |
|---|---|---|
| 2400nM (1200nM on cells) | see above | 13,2 |
| 1200nM (600nM on cells) | 1:1 in dilution medium | 6,6 |
| 600nM (300nM on cells) | 1:1 in dilution medium | 3,3 |
| 300nM (150nM on cells) | 1:1 in dilution medium | 1.65 |
| 150nM (75nM on cells) | 1:1 in dilution medium | 0,83 |
| 75nM (38nM on cells) | 1:1 in dilution medium | 0,42 |
| 38nM (19nM on cells) | 1:1 in dilution medium | 0,21 |
| 19nM (10nM on cells) | 1:1 in dilution medium | 0,1 |

Table of Serial Dilution. For generation of a dose response curve seven one-to-one serial dilution steps were performed for each sample and all dilutions were analyzed in triplicates.

## Results

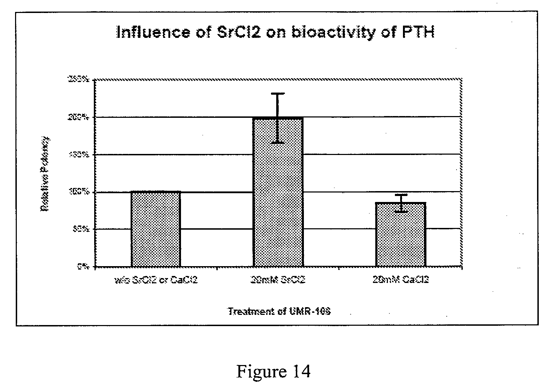

The influence of SrCl$_2$ and CaCl$_2$ on PTH bioactivity was independently monitored over five different experiments (Figure 14). These studies showed that a 24h pre-incubation of UMR-106 cells with 20mM SrC12 prior to TGplPTH treatment leads to twofold increased bioactivity, whereas CaCl$_2$ shows no change in PTH bioactivity (Figure 14). These results indicate that strontium and PTH have a synergistic effect on cAMP production and support the conclusion that a combination of PTH and strontium would lead to anabolic bone *formation in vivo.*

## Claims

1. A composition comprising fibrinogen, thrombin and a strontium-containing compound for use in bone healing or bone growth, wherein strontium is present in a 25% strontium substituted calcium salt, optionally wherein said composition is resorbed and replaced with tissue during the healing process.

2. The composition for use according to claim 1, wherein the composition is in a gel, putty, paste or liquid form.

3. The composition for use according to claims 1 or 2, further comprising a plasticizer.

4. The composition r for use according to claim 3, wherein the plasticizer is an iodine-containing compound.

5. The composition for use according to claim 4, wherein the iodine-containing compound is selected from the group consisting of diatrizoate, iodecol, iodixanol, iofratol, iogulamide, iohexol, iomeprol, iopamidol, iopromide, iotrol, ioversol, ioxagulate and metrizamide and mixtures thereof and polyvalent alcohols.

6. The composition for use according to any of the preceding claims, wherein the composition contains one or more extracellular proteins selected from the group consisting of extracellular matrix proteins such as fibronectin, cellular associated proteins, plasma derived proteins such as blood clotting factor XIII, proteases and protease inhibitors.

7. The composition for use according to any of the preceding claims, wherein the composition further comprises a clotting inducing agent such as protamine, snake venom, transglutaminases, FXIIa, or a physiologically acceptable alkaline buffer system.

8. The composition for use according to any of the preceding claims, wherein the strontium-containing compound is in a soluble microparticulate, granular form or in a solid form.

9. The compositions for use according to claim 8, wherein the strontium-containing compound is made by adding strontium into a calcium-containing compound to produce a material that comprises calcium and strontium-containing salts.

10. The composition for use according to claim 9, wherein said calcium-containing compound is a calcium phosphate.

11. The composition for use according to claim 10, wherein the calcium phosphate is selected from the group consisting of tricalcium phosphate, alpha-tricalcium phosphate, beta-tricalcium phosphate, polymorphs of calcium phosphate, hyroxyapatite, calcium carbonate, calcium sulfate and combinations thereof.

12. The composition for use according to claim 9, wherein the strontium-containing or calcium-containing compounds have a particle dimension ranging from 100 nanometers to 50 millimeters and preferably wherein the strontium-containing or calcium-containing compounds have a particle dimension of 0.5 to 5 millimeters.

13. The composition r for use according to any of the preceding claims, wherein the strontium-containing compound is selected from the group consisting of strontium chloride, strontium ranelate, strontium acetate, strontium glutamate, strontium aspartate, strontium malonate, strontium maleate, strontium ascorbate, strontium threonate, strontium lactate, strontium phosphate, strontium apatites, strontium pyruvate, strontium alpha-ketoglutarate and strontium succinate.

14. The composition for use according to any of the preceding claims, further comprising a strontium co-ligand selected from the group consisting of groups calcimimetics, osteocalcin, amino acids such as L-arginine, and arginomimetics and arginine analogs.

15. The composition for use according to claim 3, wherein the plasticizer is a polyethylene glycol, a polyvalent alcohol, glycerol, or sugar selected from the group consisting of monosaccharides, disaccharides, trisaccharides, polysaccharides, and combinations thereof.

16. The composition for use according to any of the preceding claims, further comprising a protein selected from the group consisting of bone morphogenic proteins, parathyroid hormone (PTH), calcitonin, bisphosphonates, epidermal growth factor, insulinlike growth factors and TGF growth factors.

17. The composition for use according to claim 16, wherein the protein is parathyroid hormone (PTH).

18. The composition for use according to any of the preceding claims, wherein the strontium-containing compound is present in the composition at a concentration from 2.5 mM to 25 mM.

19. The composition for use according to any of the preceding claims, wherein the strontium-containing compound is present in the composition at between 30 to 50% (w/v) of the combined fibrinogen and thrombin.

20. The composition for use according to any of the preceding claims, wherein the amount of strontium is present as a chelate of strontium.

21. The composition for use according to any of claims 1 to 20 for use in injecting into cancellous bone for the treatment of diseased, injured or deficient bone in a living subject.

22. The composition for use according to any of claims 1 to 20 for use in repairing a bone defect by filling the defect in an amount effect cause repair of said defect, said use of said composition being in combination with mechanically fixating the bone or portions thereof for achieving bone repair.

23. The composition for use according to any of claims 1 to 20 for use in promoting new bone growth at the site of a bone defect.

**Patentansprüche**

1. Zusammensetzung, umfassend Fibrinogen, Thrombin und eine Strontium-enthaltende Verbindung zur Verwendung in Knochenheilung oder Knochenwachstum, wobei Strontium in einem 25% Strontium-substituierten Calciumsalz vorliegt, wobei gegebenenfalls die Zusammensetzung resorbiert und mit Gewebe während des Heilungsprozesses ersetzt wird.

2. Zusammensetzung zur Verwendung gemäß Anspruch 1, wobei die Zusammensetzung in einem Gel, Kitt, Paste oder flüssiger Form ist.

3. Zusammensetzung zur Verwendung gemäß Anspruch 1 oder 2, weiter umfassend einen Weichmacher.

4. Zusammensetzung zur Verwendung gemäß Anspruch 3, wobei der Weichmacher eine Iod-enthaltende Verbindung ist.

5. Zusammensetzung zur Verwendung gemäß Anspruch 4, wobei die Iodenthaltende Verbindung aus der Gruppe, bestehend aus Diatrizoat, Iodecol, Iodixanol, Iofratol, Iogulamid, Iohexol, Iomeprol, Iopamidol, Iopromid, Iotrol, Ioversol, Iogulat und Metrizamid und Gemischen davon und mehrwertigen Alkoholen, ausgewählt ist.

6. Zusammensetzung zur Verwendung gemäß einem der vorhergehenden Ansprüche, wobei die Zusammensetzung ein oder mehrere extrazelluläre Proteine enthält, ausgewählt aus der Gruppe, bestehend aus extrazellulären Matrixproteinen wie Fibronectin, zellulär-verknüpften Proteinen, Plasma-abgeleiteten Proteinen wie Blutgerinnungsfaktor XIII, Proteasen und Protease-Inhibitoren.

7. Zusammensetzung zur Verwendung gemäß einem der vorhergehenden Ansprüche, wobei die Zusammensetzung weiter ein Gerinnungs-induzierendes Mittel wie Protamin, Schlangengift, Transglutaminasen, FXIIa oder ein physiologisch verträgliches Alkalipuffersystem umfaßt.

8. Zusammensetzung zur Verwendung gemäß einem der vorhergehenden Ansprüche, wobei die Strontium-enthaltende Verbindung in einer löslichen mikropartikulären granulären Form oder in einer festen Form ist.

9. Zusammensetzung zur Verwendung gemäß Anspruch 8, wobei die Strontium-enthaltende Verbindung durch Zugeben von Strontium in eine Calciumenthaltende Verbindung zur Erzeugung eines Materials, welches Calcium- und Strontium-enthaltende Salze umfaßt, hergestellt worden ist.

10. Zusammensetzung zur Verwendung gemäß Anspruch 9, wobei die Calciumenthaltende Verbindung ein Calciumphosphat ist.

11. Zusammensetzung zur Verwendung gemäß Anspruch 10, wobei das Calciumphosphat aus der Gruppe ausgewählt ist, bestehend aus Tricalciumphosphat, Alphat-Tricalciumphosphat, Beta-Tricalciumphosphat, Polymorphen von Calciumphosphat, Hydroxyapatit, Calciumcarbonat, Calciumsulfat und Kombinationen davon.

12. Zusammensetzung zur Verwendung gemäß Anspruch 9, wobei die Strontium-enthaltenden oder Calcium-enthaltenden Verbindungen eine Teilchengröße im Bereich von 100 Nanometern bis 50 Millimetern aufweisen und wobei vorzugsweise die Strontium-enthaltenden oder Calcium-enthaltenden Verbindungen eine Teilchengröße von 0,5 bis 5 Millimeter aufweisen.

13. Zusammensetzung zur Verwendung gemäß einem der vorhergehenden Ansprüche, wobei die Strontium-enthaltende Verbindung ausgewählt ist aus der Gruppe, bestehend aus Strontiumchlorid, Strontiumranelat, Strontiumacetat, Strontiumglutamat, Strontiumaspartat, Strontiummalonat, Strontiummaleat, Strontiumascorbat, Strontiumthreonat, Strontiumlactat, Strontiumphosphat, Strontiumapatiten, Strontiumpyruvat, Strontium-Alpha-Ketoglutamat und Strontiumsuccinat.

14. Zusammensetzung zur Verwendung gemäß einem der vorhergehenden Ansprüche, weiter umfassend einen Strontium-Co-Ligand, ausgewählt aus der Gruppe, bestehend aus der Gruppe von Calcimimetika, Osteocalcin, Aminosäuren wie L-Arginin und Arginomimetika und Argininanalogen.

15. Zusammensetzung zur Verwendung gemäß Anspruch 3, wobei der Weichmacher ein Polyethylenglycol, ein mehrwertiger Alkohol, Glycerin oder Zucker, ausgewählt aus der Gruppe, bestehend aus Monosacchariden, Disacchariden, Trisacchariden, Polysacchariden und Kombinationen davon, ist.

16. Zusammensetzung zur Verwendung gemäß einem der vorhergehenden Ansprüche, weiter umfassend ein Protein, ausgewählt aus der Gruppe, bestehend aus Knochen-morphogenen Proteinen, Parathyroidhormon (PTH), Calcitonin, Bisphosphonaten, epidermalem Wachstumsfaktor, Insulin-artigen Wachstumsfaktoren und TGF Wachstumsfaktoren.

17. Zusammensetzung zur Verwendung gemäß Anspruch 16, wobei das Protein Parathyroidhormon (PTH) ist.

18. Zusammensetzung zur Verwendung gemäß einem der vorhergehenden Ansprüche, wobei die Strontium-enthaltende Verbindung in der Zusammensetzung bei einer Konzentration von 2,5 mM bis 25 mM vorliegt.

19. Zusammensetzung zur Verwendung gemäß einem der vorhergehenden Ansprüche, wobei die Strontium-enthaltende Verbindung in der Zusammensetzung bei zwischen 30 bis 50% (w/v) des kombinierten Fibrinogen und Thrombins vorliegt.

20. Zusammensetzung zur Verwendung gemäß einem der vorhergehenden Ansprüche, wobei die Menge an Strontium als ein Chelat von Strontium vorliegt.

21. Zusammensetzung zur Verwendung gemäß einem der Ansprüche 1 bis 20 zur Verwendung im Injizieren in Spongiosa zur Behandlung von erkranktem, verletztem oder mangelhaftem Knochen in einem lebenden Subjekt.

22. Zusammensetzung zur Verwendung gemäß einem der Ansprüche 1 bis 20 zur Verwendung beim Beheben eines Knochendefekts durch Füllen des Defekts in einer Menge, welche die Behebung des Defekts bewirkt, wobei die Verwendung der Zusammensetzung in Kombination mit mechanischem Fixieren des Knochens oder Bereichen davon zum Erreichen der Knocheninstandsetzung ist.

23. Zusammensetzung zur Verwendung gemäß einem der Ansprüche 1 bis 20 zur Verwendung in der Förderung von neuem Knochenwachstum an der Stelle eines Knochendefekts.

## Revendications

1. Composition comprenant du fibrinogène, de la thrombine et un composé contenant du strontium pour utilisation dans la cicatrisation osseuse ou l'ossification, dans laquelle le strontium est présent dans un sel de calcium substitué à 25 % par du strontium, éventuellement dans laquelle ladite composition est résorbée et remplacée par du tissu lors du processus de cicatrisation.

2. Composition pour utilisation selon la revendication 1, laquelle composition se présente sous la forme d'un gel, d'un mastic, d'une pâte ou d'un liquide.

3. Composition pour utilisation selon les revendications 1 ou 2, comprenant en outre un plastifiant.

4. Composition pour utilisation selon la revendication 3, dans laquelle le plastifiant est un composé contenant de l'iode.

5. Composition pour utilisation selon la revendication 4, dans laquelle le composé contenant de l'iode est choisi dans le groupe constitué de diatrizoate, d'iodécol, d'iodixanol, d'iofratol, d'iogulamide, d'iohexol, d'ioméprol, d'iopamidol, d'iopromide, d'iotrol, d'ioversol, d'ioxagulate et de métrizamide et de mélanges de ceux-ci et d'alcools polyvalents.

6. Composition pour utilisation selon l'une quelconque des revendications précédentes, laquelle composition contient une ou plusieurs protéines extracellulaires choisies dans le groupe constitué des protéines de la matrice extracellulaire telles que la fibronectine, des protéines associées cellulaires, des protéines dérivées du plasma telles que le facteur de coagulation du sang XIII, des protéases et des inhibiteurs de protéase.

7. Composition pour utilisation selon l'une quelconque des revendications précédentes, laquelle composition comprend en outre un agent induisant la coagulation tel que la protamine, le venin de serpent, les transglutaminases, le FXIIa ou un système de tampon alcalin physiologiquement acceptable.

8. Composition pour utilisation selon l'une quelconque des revendications précédentes, dans laquelle le composé contenant du strontium se présente sous une forme granulaire microparticulaire soluble ou sous une forme solide.

9. Composition pour utilisation selon la revendication 8, dans laquelle le composé contenant du strontium est préparé en ajoutant du strontium dans un composé contenant du calcium pour produire un matériau qui comprend des sels contenant du calcium et du strontium.

10. Composition pour utilisation selon la revendication 9, dans laquelle ledit composé contenant du calcium est un phosphate de calcium.

11. Composition pour utilisation selon la revendication 10, dans laquelle le phosphate de calcium est choisi dans le groupe constitué du phosphate de tricalcium, du phosphate d'alpha-tricalcium, du phosphate de bêta-tricalcium, des polymorphes de phosphate de calcium, de l'hydroxyapatite, du carbonate de calcium, du sulfate de calcium et des combinaisons de ceux-ci.

12. Composition pour utilisation selon la revendication 9, dans laquelle les composés contenant du strontium ou du calcium présentent une dimension des particules située dans la plage de 100 nanomètres à 50 millimètres et de préférence dans laquelle les composés contenant du strontium ou du calcium présentent une dimension des particules de 0,5 à 5 millimètres.

13. Composition pour utilisation selon l'une quelconque des revendications précédentes, dans laquelle le composé contenant du strontium est choisi dans le groupe constitué du chlorure de strontium, du ranélate de strontium, de l'acétate de strontium, du glutamate de strontium, de l'aspartate de strontium, du malonate de strontium, du maléate de strontium, de l'ascorbate de strontium, du thréonate de strontium, du lactate de strontium, du phosphate de strontium, des apatites de strontium, du pyruvate de strontium, de l'alpha-cétoglutarate de strontium et du succinate de strontium.

14. Composition pour utilisation selon l'une quelconque des revendications précédentes, comprenant en outre un co-ligand du strontium choisi dans le groupe constitué des calcimimétiques, de l'ostéocalcine, des acides aminés tels que la L-arginine, et des arginomimétiques et des analogues d'arginine.

15. Composition pour utilisation selon la revendication 3, dans laquelle le plastifiant est un polyéthylène glycol, un alcool polyvalent, le glycérol, ou un sucre choisi dans le groupe constitué des monosaccharides, des disaccharides, des trisaccharides, des polysaccharides, et des combinaisons de ceux-ci.

16. Composition pour utilisation selon l'une quelconque des revendications précédentes, comprenant en outre une protéine choisie dans le groupe constitué des protéines morphogéniques osseuses, de la parathormone (PTH), de la calcitonine, des bisphosphonates, du facteur de croissance de l'épiderme, des facteurs de croissance insulinomimétiques et des facteurs de croissance transformants (TGF).

17. Composition pour utilisation selon la revendication 16, dans laquelle la protéine est la parathormone (PTH).

18. Composition pour utilisation selon l'une quelconque des revendications précédentes, dans laquelle le composé contenant du strontium est présent dans la composition à une concentration de 2,5 mM à 25 mM.

19. Composition pour utilisation selon l'une quelconque des revendications précédentes, dans laquelle le composé contenant du strontium est présent dans la composition à une concentration située entre 30 et 50 % (en p/v) du fibrinogène et de la thrombine combinés.

20. Composition pour utilisation selon l'une quelconque des revendications précédentes, dans laquelle la quantité de strontium est présente sous la forme d'un chélate de strontium.

21. Composition pour utilisation selon l'une quelconque des revendications 1 à 20 pour utilisation par injection dans un os spongieux pour le traitement d'un os malade, blessé ou déficient chez un sujet vivant.

22. Composition pour utilisation selon l'une quelconque des revendications 1 à 20 pour utilisation dans la réparation d'un défaut osseux par remplissage du défaut en une quantité dont l'effet provoque la réparation dudit défaut, ladite utilisation de ladite composition étant combinée à la fixation mécanique de l'os ou de parties de celui-ci pour que soit réalisée la réparation osseuse.

23. Composition pour utilisation selon l'une quelconque des revendications 1 à 20 pour utilisation dans la promotion de la croissance d'os nouveau au niveau du site d'un défaut osseux.

Figure 1

Figure 2

Figure 3.

Figure 4.

Figure 5.

Figure 6

Top Surface

Top Surface

Figure 7

Figure 8:

Figure 9

Figure 10:

Figure 11

Figure 12

Figure 13

Figure 14

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5736132 A
- US 5549904 A
- US 20050119746 A, Lidgren and Nilson
- US 20080065091 A
- US 20080058828 A
- US 20070073307 A
- US 4969888 A
- US 5108404 A
- EP 1405647 A
- EP 1655042 A
- WO 9621628 A
- WO 9117777 A
- WO 9112032 A
- US 20080066495 A

**Non-patent literature cited in the description**

- **PARIKH SN.** *J. Postgrad. Med.,* 2002, vol. 48, 142-148
- **DAHL et al.** *Bone,* vol. 28 (4), 446-453
- **FERRARO et al.** *Calcif Tissue Int.,* 1983, vol. 35, 258-60
- **MEUNIER PJ et al.** *N Engl J Med.,* 2004, vol. 350 (5), 459-68
- **KENDLER.** *Curr. Osteopor. Rep.,* 2006, vol. 4 (1), 34-9
- **MARIE.** *Curr. Opin. Rheum.,* 2006, vol. 18 (1), 11-5
- **AMMANN.** *Bone,* 2006, vol. 38 (1), 15-8
- **CLOSE et al.** *Expert Opin Pharmacother,* 2006, vol. 7 (12), 1603-15
- **WONG, CHI-TAK.** Osteoconduction and osseointegration of a strontium-containing hydroxyapatite bioactive bone cement : in vitro and in vivo investigations. University of Hong Kong, 2004
- **ZHAO et al.** *J Biomed Mater Res B Appl Biomater.,* 2004, vol. 69 (1), 79-86
- **PI et al.** *J. Biol. Chem.,* 2005